# EUROPEAN PATENT SPECIFICATION

(11) **EP 0 245 855 B1**
(45) Date of publication and mention of the grant of the patent: **07.01.1993**
(21) Application number: 87106961.3
(22) Date of filing: 14.05.1987
(51) Int. Cl.: A61K 33/06, A61K 31/70, A61K 47/38

(54) **Sucralfate preparations for application on esophagus mucosa**
Sucralfatpräparate zur Anwendung für die Speiseröhrenschleimhaut
Préparations à base de sucralfate destinées à l'application sur la muqueuse de l'oesophage

(30) Priority: 16.05.1986 JP 110686/86
(43) Date of publication of application: 19.11.1987
(73) Proprietor: Chugai Seiyaku Kabushiki Kaisha, Tokyo (JP)
(72) Inventor: Ishihara, Kouji, Soka-shi Saitama-ken (JP); Ogasawara, Toshichika, Tokyo (JP); Igusa, Kazuo, Hachioji-shi Tokyo (JP)
(74) Representative: VOSSIUS & PARTNER

(56) References cited:
- EP-A- 0 136 100
- DE-A- 3 315 800
- DE-A- 3 430 809
- US-A- 3 432 489
- DEUTSCHE MEDIZINISCHE WOCHENSCHRIFT, Georg Thieme Verlag, Stuttgart, New York, 111. Jahrgang, no. 50, 1986 A.L. BLUM et al. pp. 1910-1915

## Description

This invention relates to sucralfate preparations for application on esophagus mucosa.

An aluminum salt of sucrose sulfate ester (conventionally known as sucralfate) is an excellent ulcer curative agent with minimal side effects, and is extensively used in anti-ulcer therapy.

Thus DE-A-34 30 809 describes sucralfate preparations in form of suspensions comprising 1 to 5% by weight xanthan gum and 1-12.5% by weight of a peptizer.

The subject-matter of the EP-A-0 136 100 is to provide a suspension for treatment of ulcerative conditions comprising a pharmaceutically effective amount of sucralfate and a pharmaceutically acceptable suspending agent and forming a viscous mass when added to 0.2 to 1N hydrochloric acid.

Sucralfate is characterized by anti-pepsin and anti-acid effects in that it binds to the pepsin and acids in gastric juice, which are two agents that attack the ulcer-affected area of digestive tracts, and thereby inhibits their activities directly. Sucralfate is also characterized by its ability to protect the mucosa in that it forms a sucralfate coat on the mucosa of the digestive tract and protects it from the attacking agents. These effects combine to provide sucralfate with high anti-ulcer activities. It has also been found that sucralfate is effective in revascularization and in accelerating the growth of regenerated mucosa, and hence is considered to have a great potential for use in promoting the healing of ulcers. Sucralfate has an ability to selectively bind to the ulcer-affected mucosa of the digestive tract rather than the normal mucosa of the digestive tract and form a protective coat against the invasion of attacking agents. This ability is the most characteristic feature of sucralfate, not found in other anti-ulcer treatments. The esophagus differs in function from other digestive organs, in that it is not concerned with the digestion of foods or the absorption of the nutrients; but instead plays an important role in transporting foods from the oral cavity to the intraperitoneal digestive tract. In a normal person the digestive liquid which has been regurgitated from the stomach to the esophagus may be returned immediately by the action of vermiculation of the esophagus. However, if regurgitation occurs frequently or if the regurgitated gastric juice or bile is retained in the esophagus, then regurgitational esophagitis will develop. Another type of regurgitational esophagitis, not associated with actual regurgitation symptoms, is also known. This type of esophagitis is considered to be caused by various factors which have an affect upon each other. It is also known that this type of regurgitational esophagitis repeatedly results in recrudescence and relapse of diseases, and that a long period of time is required for therapy and observation in the subsequent curing process. Further, this disease is associated with deformation and stricture of the esophagus. For these reasons, this type of esophagitis is recognized to be very difficult to cure and has been mainly treated by surgical means.

In the primary stage of the disease, nonserious regurgitational esophagitis may usually be cured by alimentary therapy and/or by controlling the patient's daily routine. However, if the disease is more serious or is associated with other diseases, simple alimental therapy is not sufficient to cure the disease effectively and a more positive treatment using drugs is required.

In case it is intended that a pharmaceutical preparation be orally administered and adhered on inflammatory sites in wet tissue such as those formed in esophageal mucosa, the administration of a preparation in the form of tablet, troche or sublingual tablet is unable to readily provide such desired effects as binding or adhering of the pharmaceutical preparation to the inflammatory sites because a patient often swallows or crunches the solid preparation. These problems can not be solved by simply increasing the dose level of the drug. On the other hand, the administration of an effective component in the form of powder will not provide the desired effects for several reasons, typically because the administration of a powdery preparation is associated with pain.

On the other hand, a preparation in the form of a suspension has advantages in that the dose level can be easily controlled, and in that it is a very easy form for oral dosage. Under these circumstances, the inventors of this application extensively studied the dosage form of sucralfate in order to fully develop its above-mentioned advantages by the use of a suspension dosage form. In general, it has been believed that from the practical and organoleptic viewpoints, a suspension having a viscosity of higher than 500 mPas [centipoise (cP)] is difficult to use clinically, and that it is preferable to use a suspension with a viscosity of lower than 50 mPas (cP), especially from the organoleptic viewpoint. With this knowledge, the inventors formulated various types of sucralfate preparations with less than 500 mPas (cP) at 20°C. These preparations include suspensions without any suspension stabilizer or thickener; suspensions containing a water-insoluble or water soluble polymeric substance as a suspension stabilizer or thickener; suspensions containing a thickener such as glycerine, sucrose or sorbitol; suspensions formulated by using sucralfate having particles of predetermined and relatively uniform sizes; suspensions containing various additives such as sugars, salts, acids or bases; suspensions having various concentrations of suspended particles; suspensions containing a surfactant for the purpose of changing their physical properties; suspensions prepared by varying the conditions of a homogenizing mixer; and suspensions using the above-mentioned conditions with a different suspension medium. These suspensions were tested with respect to their selective binding to the ulcerated or inflammatory sites of esophageal mucosa. The suspension was forcefully administered through a probe into the esophagus of rats in which esophagitis had been artificially induced or which have not been treated. The selective binding of sucralfate to ulcerated or inflammatory sites was determined by measuring the amount of sucrose sulfate and aluminum adhered to each of the ulcerated or inflammatory sites as well as sites which had not been ulcerated or were not inflammatory with respect to the resected pieces of mucosa having the same and predetermined size. Three hours after the adminstration of sucralfate, the rats were killed and the pieces of mucosa were prepared. The amount of each of aluminum and sucrose adhered on the samples was determined by the methods of Okamura et al., Bull. Chem. Soc. Japan, 31,783 (1958) and the method of Nagashima et al., "Arzneim-Forsch. 29, 1668 (1979)", respectively.

The results of the above-mentioned tests showed that no sucralfate was selectively adhered to any of the inflammatory sites with any of the formulated suspensions. It is believed that this phenomenon is due to secretions such as saliva washing off the suspension administered. Since it is impossible to completely eliminate this phenomenon, the inventors confirmed that none of the suspensions having the above-mentioned viscosity is suitable as a preparation for oral administration.

Under this situation, the inventors continued their studies and unexpectedly found that a suspension containing sucralfate and having a viscosity in the range of from 1,000 to 5,000 mPas (cP) at 20°C most effectively adheres on the inflammatory sites and this invention was thereby completed. According to their further studies, the inventors found that the desired effects are not achieved if substances having low molecular weights such as sorbitol, sucrose or glycerin are used for formulating a sucralfate-containing suspension in order to increase the viscosity of the suspension to a desired range.

In order to ensure that sucralfate effectively adheres to inflammatory sites, it is necessary that it is suspended in an aqueous solution or suspension of one or more water-soluble or water-insoluble, natural or synthetic or semi-synthetic polymeric substances and having a viscosity of from 1,000 to 5,000 mPas (cP).

Thus the present invention relates to a sucralfate preparation in the form of a suspension comprising sucralfate suspended in an aqueous solution or suspension of one or more water-soluble or water-insoluble cellulose substances as a suspending agent or thickening agent characterized in that the cellulose suspending agent or thickening agent comprises at least one of crystalline cellulose, hydroxyethylcellulose or hydroxypropylmethylcellulose and which is added in an amount that the suspension has a viscosity of from 1,000 to 5,000 mPas (centipoise) at 20°C.

The cellulose substances which are useful in this invention include water-soluble cellulose substances such as crystalline cellulose, carboxymethylcellulose and calcium carboxymethylcellulose, methylcellulose (substitution degree: 1.6 - 2), ethylcellulose (substitution degree: 1 - 1.5), propylcellulose, hydroxymethylcellulose, hydroxyethylcellulose, hydroxypropylcellulose, hydroxyethylmethylcellulose, hydroxyethylpropylcellulose, hydroxypropylmethylcellulose, carboxymethyl-starch, carboxymethylethylcellulose and carboxymethylhydroxyethylcellulose. These cellulose substances can be used alone or in admixture. Hydroxyethylcellulose and hydroxypropylmethylcellulose are especially preferable for providing the desired effects of this invention. The amount of polymer to be used in this invention may be determined based on the specific polymer used and the viscosity of the suspension. In addition, if desired, one or more appropriate additive, such as sweeteners, flavoring, preservatives or excipients can be incorporated in the suspension of this invention. These additives may be used by selecting the species, amount, and incorporation manner in accordance with conventional practice for formulation of pharmaceutical preparations.

The invention is further illustrated by the following Examples which do not limit the invention.

### Example 1

Crystalline cellulose (sold under the trade name of Avisel RC-591NF: 10 g) was preliminarily dispersed in distilled water in an agitating and homogenizing mixer. To the dispersion was slowly added DL-alanine (5 g) and then sucralfate (50 g) while stirring. After a preservative (0.4 g) had been added and the mixture stirred, water was added to bring the total volume to 500 ml to give a suspension which was used as a test sample.

The process as described above was repeated except that crystalline cellulose was not used, thus producing a suspension to serve as the control.

### Example 2

Hydroxypropylmethylcellulose (7.5 g) was preliminarily dispersed in distilled water in an agitating and homogenizing mixer. To the dispersion was slowly added DL-alanine (5 g) and then sucralfate. After further adding a 70% D-sorbitol aqueous solution (25 g) and a preservative (0.4 g) to the mixture and stirring it, the mixture was treated as in Example 1 to give a suspension which was used as a test sample.

Separately, the method described above was repeated except that hydroxypropylmethylcellulose was not used, thus producing a suspension to serve as a control.

### Example 3

Hydroxyethylcellulose (10 g) was dissolved in water. To the solution was slowly added DL-alanine (5 g) and, then, sucralfate (50 g) while stirring in a mixer. Then, sucrose (25 g) and a preservative (0.4 g) were added to the mixture which was treated as shown in Example 1 to give a suspension which was used as a sample of this invention.

Separately, the process described above was repeated except that hydroxyethylcellulose was not used, thus producing a suspension to serve as a control.

The experimental induction of regurgitational esophagitis has been tried. One of the known methods for inducing esophagitis comprises causing endogenous regurgitation by forming esophagocele but does not give constant results. Therefore, the inventors employed a method in which Wistar-strain rats were used as test animals and an acid was injected in a lower part of the esophagus to induce esophagitis.

There is a criterion prepared by the Association of Esophageal Disease in Japan with respect to diagnosis for esophagitis on the basis of the histological view. The esophagitis induced in the test animals by the inventors was found to develop changes of tissue corresponding to those defined in the criterion. These were various cellular infiltrations in mucosa or submucosa, cellular infiltration of intrinsic muscle layer, or hemostatis or dilation of blood vessels in submucosa which often develop during the induction of esophagitis.

In accordance with these findings, the inventors conducted the following experiment.

### Experiment

Wistar-strain male rats weighing about 200 g were caused to fast for 48 hours, their abdomen opened, and pylorus ligated. In order to relax the tension of sphincter at a lower part of the esophagus, atropine (0.2 mg/kg) and then 2 ml of 1N hydrochloric acid were orally administered by use of a cannula to develop esophagitis with remarkable degeneration of mucosal epithelium, hemostatis and cellular infiltration. These tissue degenerations clearly developed about one hour after the treatment.

The sample or control which was prepared by each of the Examples was forcefully administered to the esophagitis-induced rats through a cannula. In this treatment, the test animals were divided into groups of six members each and the sample or control was administered in a dose of 0.5 ml per head in terms of suspension, or 50 mg per head in terms of sucralfate. Three hours after the administration, the rats were killed and the quantity of each of aluminum and sucrose sulfate ester adhered to the inflammatory sites of esophagitis was determined.

The results are shown in Table 1 below. As is clear from the test results, the values of aluminum and the ester with respect to the sample suspension were larger than those of the control.

**Table 1**

| Examples | Test suspension | Viscosity (cP, 20°C) mPas | Sucrose sulfate ester (µmol/cm²) | Aluminum (µmol/cm²) |
|---|---|---|---|---|
| 1 | sample | 1080.0 | 1.458 | 2.458 |
| | control | 7.5 | 0.066 | 0.115 |
| 2 | sample | 1502.5 | 1.619 | 2.421 |
| | control | 8.3 | 0.019 | 0.043 |
| 3 | sample | 2030.0 | 1.589 | 2.356 |
| | control | 10.6 | 0.008 | 0.017 |

## Claims (Claims for the following Contracting State(s): BE, CH, DE, FR, GB, IT, LI, NL, SE)

1. A sucralfate preparation in the form of a suspension comprising sucralfate suspended in an aqueous solution or suspension of one or more water-soluble or water-insoluble cellulose substances as a suspending agent or thickening agent characterized in that the cellulose suspending agent or thickening agent comprises at least one of crystalline cellulose, hydroxyethylcellulose or hydroxypropylmethylcellulose and which is added in an amount that the suspension has a viscosity of from 1000 to 5,000 mPas (centipoise) at 20° C.

2. A sucralfate preparation according to Claim 1 for use in the treatment of inflammations of esophageal mucosa.

## Claims (Claims for the following Contracting State(s): AT, ES)

1. A method for preparing a sucralfate preparation in the form of a suspension which comprises suspending sucralfate in an aqueous solution or suspension of one or more water-soluble or water-insoluble cellulose substances as a suspending agent or thickening agent, characterized in that the cellulose suspending agent or thickening agent comprises at least one of crystalline cellulose, hydroxyethylcellulose or hydroxypropylmethylcellulose and which is added in an amount that the suspension has a viscosity of from 1,000 to 5,000 mPas (centipoise) at 20° C.

2. A method for preparing a sucralfate preparation according to Claim 1 for use in the treatment of inflammations of esophageal mucosa.

## Patentansprüche (Patentansprüche für folgende(n) Vertragsstaat(en): BE, CH, DE, FR, GB, IT, LI, NL, SE)

1. Sucralfatzubereitung in Form einer Suspension, umfassend Sucralfat, das in einer wäßrigen Lösung oder Suspension einer oder mehrerer wasserlöslicher oder wasserunlöslicher Cellulosesubstanzen als Suspendier- oder Verdickungsmittel suspendiert ist, dadurch gekennzeichnet, daß das Cellulosesuspendiermittel oder -verdickungsmittel mindestens eine der Substanzen kristalline Cellulose, Hydroxyethylcellulose oder Hydroxypropylmethylcellulose umfaßt, und das in einer Menge zugegeben wird, daß die Suspension eine Viskosität von 1.000 bis 5.000 mPas (Zentipoise) bei 20°C besitzt.

2. Sucralfatzubereitung gemäß Anspruch 1 zur Verwendung bei der Behandlung von Entzündungen der ösophagealen Mucosa.

## Patentansprüche (Patentansprüche für folgende(n) Vertragsstaat(en): AT, ES)

1. Verfahren zur Herstellung einer Sucralfatzubereitung in Form einer Suspension, umfassend das Suspendieren von Sucralfat in einer wäßrigen Lösung oder Suspension einer oder mehrerer wasserlöslicher oder wasserunlöslicher Cellulosesubstanzen als Suspendier- oder Verdickungsmittel, dadurch gekennzeichnet, daß das Cellulosesuspendiermittel oder -verdickungsmittel mindestens eine der Substanzen kristalline Cellulose, Hydroxyethylcellulose oder Hydroxypropylmethylcellulose umfaßt, und das in einer Menge zugegeben wird, daß die Suspension eine Viskosität von 1.000 bis 5.000 mPas (Zentipoise) bei 20°C besitzt.

2. Verfahren zur Herstellung einer Sucralfatzubereitung gemäß Anspruch 1 zur Verwendung bei der Behandlung von Entzündungen der ösophagealen Mucosa.

## Revendications (Revendications pour l'(les) Etat(s) contractant(s) suivant(s): BE, CH, DE, FR, GB, IT, LI, NL, SE)

1. Préparation à base de sucralfate sous la forme d'une suspension comprenant du sucralfate en suspension dans une solution ou suspension aqueuse d'une ou plusieurs substances cellulosiques solubles dans l'eau ou insolubles dans l'eau comme agent de mise en suspension ou agent épaississant, caractérisée en ce que l'agent de mise en suspension ou agent épaississant cellulosique comprend au moins l'une d'entre la cellulose cristalline, l'hydroxyéthylcellulose et l'hydroxypropylméthylcellulose qui est ajoutée en une quantité telle que la suspension ait une viscosité de 1000 à 5000 mPas (centipoises) à 20°C.

2. Préparation à base de sucralfate suivant la revendication 1, à utiliser pour le traitement des inflammations de la muqueuse de l'oesophage.

## Revendications (Revendications pour l'(les) Etat(s) contractant(s) suivant(s): AT, ES)

1. Procédé pour la production d'un préparation à base de sucralfate sous la forme d'une suspension qui comprend la suspension du sucralfate dans une solution ou suspension aqueuse d'une ou plusieurs substances cellulosiques solubles dans l'eau ou insolubles dans l'eau comme agent de mise en suspension ou agent épaississant, caractérisée en ce que l'agent de mise en suspension ou agent épaississant cellulosique comprend au moins l'une d'entre la cellulose cristalline, l'hydroxyéthylcellulose et l'hydroxypropylméthylcellulose qui est ajoutée en une quantité telle que la suspension ait une viscosité de 1000 à 5000 mPas (centipoises) à 20°C.

2. Procédé pour la production d'un préparation à base de sucralfate suivant la revendication l, à utiliser pour le traitement des inflammations de la muqueuse de l'oesophage.
